# EUROPEAN PATENT SPECIFICATION

(11) **EP 3 079 615 B1**
(45) Date of publication and mention of the grant of the patent: **10.04.2019**
(21) Application number: 14816007.0
(22) Date of filing: 24.11.2014
(51) Int. Cl.: A61B 17/80

(54) **BONE PLATE**
KNOCHENPLATTE
LAME OSSEUSE

(30) Priority: 11.12.2013 US 201361914526 P; 11.12.2013 US 201314103285
(43) Date of publication of application: 19.10.2016
(73) Proprietor: DePuy Synthes Products, Inc., Raynham, Massachusetts 02767-0350 (US)
(72) Inventor: WOLF, Stefan, CH-4528 Zuchwil (CH); AEBI, This, CH-4528 Zuchwil (CH)
(74) Representative: Klunker IP Patentanwälte PartG mbB
(86) International application number: PCT/US2014/067104
(87) International publication number: WO 2015/088760

(56) References cited:
- EP-A1- 0 684 017
- EP-A1- 2 559 394
- WO-A1-2012/050424
- WO-A2-2004/045389
- US-A- 5 022 544
- US-A1- 2004 097 937
- US-A1- 2008 140 127
- US-A1- 2010 249 850
- US-A1- 2011 004 252
- US-A1- 2013 096 629

## Description

### FIELD OF THE INVENTION

The present invention relates to the field of surgical devices. Specifically, the invention relates to a bone plate for fixing bone segments, in particular, the proximal femur of a patient.

### BACKGROUND

For stabilizing and fixing bone fractures it is known to employ bone plates which are fixed on the bone by means of suitable fastening elements such as, for example, bone screws, along with pins, bolts or other bone anchors. To guarantee a secure fixation of the bone fragments to each other and of the bone plate on the bone, it is required that the bone anchors are incorporated into the bone precisely and so as to be adapted to the anatomy.

However, a bone plate attached to a bone is subject to mechanical loads, in particular a bending load. This load acts particularly on the weakest regions of a bone plate, namely those regions where through holes are provided. In particular, an elongate bone-plate shaft portion that is provided with through holes along its length has an irregular moment-of-resistance curve along its length and, upon excessive load, will be most likely to break in the region of a through hole, or at least be deformed in the region of a through hole, which would impede the introduction of a bone anchor through the through hole. For example, in a bone plate for the proximal femur comprising an elongate shaft portion and a head portion that is shorter and wider in comparison thereto, an especially high load occurs in the region of the through hole that is closest to the head portion, so that the risk of breakage is highest here. The object of the present invention is to provide a bone plate having a reduced risk of breakage upon a bending load on the bone plate.

US 2010/0249850 A1 discloses a bone plate having a plurality of single recesses on both surfaces. The recesses extend from the side edges into the surfaces and are disposed in an offset manner along both edges of the bone plate. Another similar bone plate having recesses along the edges of the upper surface of the bone plate that faces away from bone is disclosed in US 2004/0097937 A1. Likewise, US 2011/004252 A1 discloses a bone plate having different recesses disposed on the upper surface of the bone plate.

Various bone plates having different types of recesses or cavities that may be associated with bone fixation holes of the bone plate are disclosed in US 2013/096629 A1, WO 2004/045389 A2, US 2008/140127 A1, WO 2012/050424 A1, EP 2 559 394 A1 or EP 0 684 017 A1. The described cavities may have different functions, e.g. allowing insertion of bone screws at different angles.

### SUMMARY OF THE INVENTION

The invention is defined in independent claim 1. Preferred embodiments are defined in the dependent claims. According to an aspect of the invention, the bone plate again has a first, surface which, when the plate is positioned over the bone, faces the bone, and a second surface opposing the first surface to face away from the bone when the plate is positioned over the bone. The bone plate comprises an elongate shaft portion with two longitudinally extending side walls connecting edges of the first and second surfaces to define a width of the shaft portion (i.e., a distance between the two side walls), as well as a head portion that is shorter and wider in comparison thereto. In the shaft portion, according to this further aspect of the invention, a plurality of through holes extend through the bone plate from the first to the second surface, which are configured to respectively receive a bone anchor.

In one exemplary embodiment, the first, bone-facing surface may have recesses which extend from the opposing side surfaces over a part of the width of the shaft portion transversely to the direction of longitudinal extension of the shaft portion, each of the plurality of through holes having a plurality of recesses associated therewith, of which at least two, preferably three and, where applicable, all, recesses differ from each other and which respectively form a group of recesses, these groups of recesses being identical for each of the plurality of through holes.

Through the special arrangement of the recesses it is achieved that a bending load is distributed uniformly along the length of the shaft portion, so that a region in which a through hole is arranged does not constitute the weakest region, which, as explained at the outset, could lead to a deformation of the through hole or even to a breakage of the bone plate in the region of the through hole. Through the provision of different recesses which, respectively grouped, are associated with one of the through holes, the distribution of the bending load can be improved in comparison to identical recesses distributed over the shaft.

According to one exemplary embodiment, each group of recesses comprises a first, second and third recess which are arranged side by side in the longitudinal direction of the bone plate, the second recess lying between the first and third recesses and being formed smaller than the first and third recesses. On other words, the smaller second may have, in a plan view, a smaller dimension in at least one direction than the first and third recesses. For example, the second recess may extend a smaller distance from one of the longitudinally extending side walls of the bone plate toward a center axis of the bone plate than the first and third recesses, e.g., the second recess is shorter, and/or has a smaller dimension along the side surface, i.e. In another exemplary embodiment, the second recess may be flatter than the first and third recesses (e.g., the second recess may have a smaller depth than the first and third recesses).

In a further exemplary embodiment, each group comprises a fourth recess which is different from the other three recesses and connects the second recess to the first or third recess. The fourth recess may have a substantially quadrangular shape and is flatter (e.g., has a smaller depth) and shorter (e.g., extends a smaller distance from one of the longitudinal side walls toward the center of the bone plate) than the first, second and third recesses.

According to a further exemplary embodiment, the groups of recesses are arranged offset from each other on the opposing side surfaces of the bone plate along the longitudinal direction of the bone plate, i.e. two groups of recesses do not lie opposite each other directly on the opposing side surfaces. Preferably, the groups of recesses extend alternately from the opposing side surfaces. Thus, the distances between the groups on the respective side surfaces are not too great. Preferably, two groups of recesses following each other in the direction of longitudinal extension of the shaft portion lie opposite each other on the opposing side surfaces. However the longitudinal extensions of the two group of recesses intersect, i.e. before one group of recesses ends in the direction of longitudinal extension of the shaft portion, another group of recesses already begins on the opposing side edge. It is preferable here that recesses of mutually opposing groups of recesses do not pass into each other. But it is not excluded that individual - not all - recesses of a group of recesses and recesses of the opposing side surfaces may extend into each other. Preferably, the through holes are arranged offset from each other alternately with regard to a center axis of the bone plate extending in the direction of longitudinal extension of the shaft portion. In particular, a group of recesses on one side of the center axis can then be associated with a through hole that lies closer, with regard to the center axis, to the side edge of the shaft portion opposing the group of recesses.

According to a further exemplary embodiment, the recesses extend from the side surfaces at most up to a center axis of the bone plate extending in the direction of longitudinal extension of the shaft portion. In particular, it is advantageous when the recesses do not extend over the total width of the bone plate. Thus, the bone plate is not excessively weakened.

According to a further exemplary embodiment, the bone plate has a head portion which is shorter and wider than the shaft portion, with the through holes and the recesses being arranged in the shaft portion. A cross-sectional area of the shaft portion transversely to the direction of longitudinal extension of the shaft portion is substantially constant along the longitudinal direction. In other words, a distance between side walls of the bone plate and a distance between the first and second surfaces of the bone plate are constant along a length of the shaft portion of the bone plate. The size and arrangement of the recesses correspond a distance between the through holes and the side walls, i.e. the more material of the bone plate is already lacking in a cross section due to a through hole, the smaller a recess is in this cross section, and vice versa.

In another exemplary embodiment, the shaft portion possesses along the direction of longitudinal extension of the shaft portion a substantially constant section modulus, in particular bending section modulus, in the direction of longitudinal extension. In particular, the cross-sectional area of the shaft portion along its longitudinal extension is preferably so adapted that the section modulus is constant, whereby the calculation of the section modulus includes the area moment of inertia of the cross-sectional area.

It will be appreciated that the section modulus can be subject to fluctuations due to slight deviations in the cross section or in the material, and is possibly not completely constant over the total length of the shaft portion. However, there is preferably obtained at least a uniform section modulus, i.e. the section modulus changes along the direction of the longitudinal extension of the shaft portion by at most 30%, preferably at most 20%, particularly preferably at most 10%.

In one exemplary embodiment, the recesses have their maximum depth along the longitudinally extending side surfaces. In a further exemplary embodiment, at least a respective one - and preferably all - of the recesses in the groups of recesses possess a depth that decreases from the corresponding side surface in the direction of a center axis of the bone plate extending in the direction of longitudinal extension of the shaft portion. It is advantageous when the recesses taper off as flatly as possible in the direction of the center axis, i.e. preferably enclose with the first surface an angle up to at most 20°, further preferably up to at most 15°, most preferably up to at most 10°.

According to another aspect of the invention, the bone plate includes a first surface which, when the bone plate is positioned over a bone, faces the bone, and a second surface opposing the first surface so that when the bone plate is positioned over the bone, faces away from the bone. The bone plate comprises an elongate shaft portion including two lateral side walls which extend longitudinally relative to the bone plate to connect edges of the first and second surfaces, and a head portion that is shorter (i.e., a length of the head portion is smaller than a length of the shaft portion) and wider (i.e., a distance between opposing side walls of the head portion is greater than a distance between the side walls of the shaft portion) in comparison thereto. In the shaft portion a plurality of through holes extend through the bone plate from the first to the second surface, each of which are configured to receive a bone anchor. In an exemplary embodiment, the second surface facing away from the bone may have a cavity or indentation which extends between a one of the through holes situated closest to the head portion, (e.g., a first through hole along the shaft portion), and the head portion. In one exemplary embodiment, the cavity may extend into the head portion.

As explained at the outset, the risk of breakage upon a bending load on the bone plate is greatest in the region of the first through hole. Through the provision of the cavity in a region of the non-bone-facing second surface of the bone plate in which region the head portion passes into the shaft portion, (i.e., in the region of the plate between the head portion and the first through hole), the bone plate is weakened in a targeted manner. In other words, the flexibility of the bone plate is increased in this region to relieve stress in the region of the first through hole and reduce the risk of breakage in this region.

According to another aspect, the second surface facing away from the bone has a depression which overlaps the through hole situated closest to the head portion. In one exemplary embodiment, the depression may surround at least a portion of the through hole. This depression preferably serves to reduce stress peaks upon a bending load on the bone plate in the region of the through hole. In an exemplary embodiment, the cavity and the depression border on each other and pass into each other. In other words, the cavity and the depression are open to one another. In a further exemplary embodiment, the cavity and the depression have the same dimension in a transverse direction of the bone plate at a place where they border on each other (i.e., where the cavity and the depression meet). This simplifies the production of the cavity and the depression so that the cavity and the depression may be created in a single step during manufacturing, and avoids stress peaks in a transition region. In a plan view, an end of the cavity facing the head portion may have a rounded shape.

According to one exemplary embodiment, the cavity possesses a greater dimension in a longitudinal direction of the bone plate than in a transverse direction of the bone plate. Through the elongate shape, a greater region between the first through hole and the head portion can be spanned by the cavity. In one exemplary embodiment, the cavity is spaced from the longitudinal extending side walls of the bone plate. In other words, the cavity does not extend completely in a transverse direction of the bone plate between the two side edges, but rather the full thickness (i.e., a distance between the first and second surfaces) of the bone plate is retained proximate the side walls so as not to excessively weaken the bone plate in this region.

According to one exemplary embodiment, the cavity has a curved cross-sectional profile in a direction from the shaft portion to the head portion. Preferably, a depth of the cavity increases continuously in a direction from the shaft portion to the head portion and decreases continuously after a deepest point is reached. Thus, the cavity preferably does not have a planar bottom surface, but rather a bulged shape. The deepest point of the cavity can be located, for example, approximately in the middle of the cavity. In a further exemplary embodiment, the cavity may taper as it extends toward the head portion so that an edge of the cavity is preferably formed as smoothly as possible in the direction of the head portion. For example, the cavity may form an angle smaller than 20° with respect to the second surface and, preferably smaller than 15°, particularly preferably smaller than 10°.

A depth of the cavity can be chosen based on a geometry of the bone plate so as to optimize the curve of the bending load on the bone plate. In one exemplary embodiment, the depth of the cavity amounts to up to 15% of a thickness of the bone plate, preferably up to 20%, most preferably up to 30%, of the thickness of the bone plate. The thickness of the bone plate is defined here by a cross-sectional profile, cutting the through hole, of the bone plate in a transverse direction of the bone plate between the first and second surfaces. In other words, the thickness of the bone plate is defined as a distance between the first and second surfaces.

According to one exemplary embodiment, the through holes are formed as combination holes and have a variable-angle hole portion and a compression hole overlapping therewith. The variable-angle portion has an inner surface with a thread-like structure which is interrupted by recesses and preferably tapers in the direction of the first surface. This enables an advantageous locking of a head locking screw with a head thread in the through hole. The compression hole is formed elongately in the direction of longitudinal extension of the shaft portion and possesses an unthreaded inner surface tapering in the direction of the first, bone-facing surface. The compression hole is preferably configured to receive a compression screw having a smooth screw head, the screw head sliding on the tapering inner surface of the compression hole, while the screws are being screwed in, causing a compression of the bone fracture as would be understood by one of ordinary skill in the art.

### BRIEF DESCRIPTION OF THE DRAWINGS

The preceding summary of the invention as well as the following description of an exemplary embodiment will become more easily understandable in the light of the attached drawings. An exemplary embodiment of the invention will be described hereinafter with reference to the accompanying drawings. It will be appreciated, however, that the application is not limited to the exemplary embodiment shown.
Fig. 1 shows a top plan view of a bone plate, according to an exemplary embodiment of the present invention;
Fig. 2 shows a side view of the bone plate of Fig. 1;
Fig. 3 shows a bottom plan view of the bone plate of Fig. 1;
Fig. 4 shows a perspective view of a portion of the bone plate of Fig. 1;
Fig. 5 shows a perspective view of a portion of the bone plate of Fig. 1;
Fig. 6 shows an enlarged detailed view of the bone plate of Fig. 1,
Fig. 7 shows an enlarged detailed view of the bone plate of Fig. 3,
Fig. 8 shows a top plan view of a through hole extending through the bone plate of Fig. 1;
Fig. 9 shows a side view of the bone plate of Fig. 1, in the region of the through hole;
Fig. 10 shows a cross-sectional view of the bone plate of Fig. 1 along the line A-A in Fig. 6;
Fig. 11 shows a cross-sectional view of the bone plate of Fig. 1 along the line B-B in Fig. 6;
Fig. 12 shows a side view of a portion of the bone plate of Fig. 1;
Fig. 13 shows a cross-sectional view of the shaft portion of the bone plate of Fig. 1, along the line C-C in Fig. 7; and
Fig. 14 shows a side view of the bone plate of Fig. 1, rotated by 10°.

### DETAILED DESCRIPTION

The present invention may be further understood with reference to the following description and the appended drawings, wherein like elements are referred to with the same reference numerals. Exemplary embodiments of the present invention describe a bone plate for fixing fractures. In particular, the exemplary embodiments describes a bone plate including a depression extending through the bone plate and surrounding a through hole extending therethrough to reduce stress peaks, for example, along a portion of the between a through hole and a lateral side wall of the bone plate. Although the exemplary embodiments specifically describe a bone plate configured for the fixation of a proximal femur, it will be understood by those of skill in the art that the bone plate of the present invention may be adapted for the fixation of any of a variety of bones and, in particular, load bearing long bones.

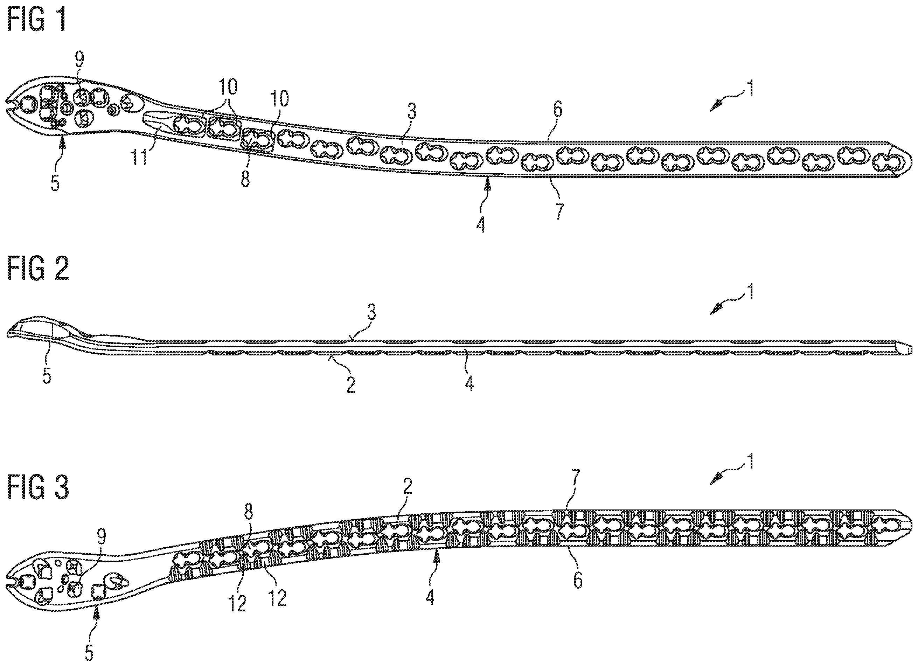

Fig. 1 shows an exemplary embodiment of a bone plate 1 in plan view from an upper side - i.e., a first surface 3 of the bone plate 1 which, when the bone plate is implanted or positioned over a bone, faces away from the bone. The represented bone plate 1 is dimensioned for treating fractures of the left proximal femur, for example, of a human patient. It will be appreciated that the concepts described hereinafter are also applicable to other bone plates, in particular to a bone plate for the right proximal femur, the distal femur, or for other long bones , such as, for example, the tibia. In Fig. 2, the bone plate 1 is represented in a side view. Fig. 3 shows the bone plate 1 from an underside of the plate, i.e., from a second surface 2 which, when the bone plate is implanted or positioned over a bone, faces the bone.

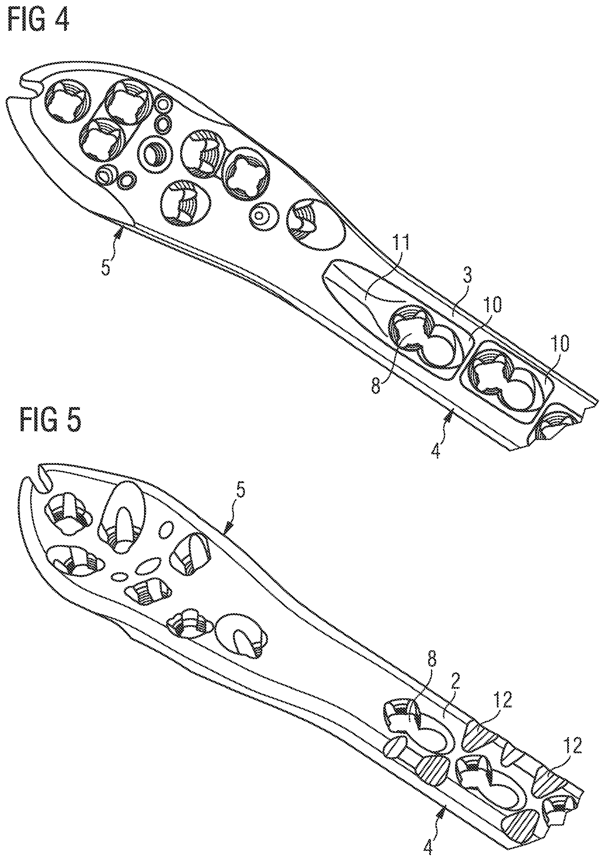

The bone plate 1 comprises an elongate shaft portion 4 and a head portion 5 along with lateral side walls 6, 7 extending along the shaft portion 4 between the surfaces 2, 3 to define a width of the shaft portion 4. The width of the shaft portion 4 is substantially constant over a total length of the shaft portion 4. The head portion 5 is wider than the shaft portion 4, i.e., a distance between the surfaces 2, 3 is larger at the head portion 5 than at the shaft portion 4, and shorter than the shaft portion 4, i.e., a length of the head portion 5 along a longitudinal axis of the bone plate 1 is smaller than a length of the shaft portion 4 along the longitudinal axis of the bone plate. In one exemplary embodiment, the head portion 5 is preferably configured to adjoin the proximal femur, in particular, the greater trochanter. For example, the head portion 5 may have a curvature corresponding to the greater trochanter, as seen especially well in Fig. 2. The head portion 5 has a plurality of through holes 9, which may be formed here as variable-angle through holes and/or fixed angle through holes. In one exemplary embodiment, the head portion 5 may include, for example, seven through holes 9, three of which may be fixed angle through holes and the remaining through holes formed as variable angle through holes. It will be understood by those of skill in the art, however, that the plurality of through holes 9 may include any combination of variable angle and fixed angled through holes. The head portion 5 is also represented in Figs. 4 and 5. Fig. 4 shows the head portion 5 in a perspective view from the first surface 3 of the bone plate 1, whereas Fig. 5 shows the head portion 5 of the bone plate 1 in a perspective view from the second surface 2 of the bone plate 1. The shaft portion 4 has an elongate shape and may include a slight curvature to correspond to the shape of the femur. It will be understood by those of skill in the art that a bone plate configured for the right proximal femur may have an accordingly opposite curvature.

The shaft portion 4 includes a plurality of through holes 8 along its length. The through holes 8 are preferably distributed uniformly along a longitudinal extension of the shaft portion 4. Preferably, the through holes 8 are arranged alternately offset from each other with respect to a center axis extending along the shaft portion 4, as seen in particular in Figs. 1 and 3. The offset arrangement of the through holes 8 in offset manner reduces the risk of the subjacent bone splitting while bone screws are being screwed into the through holes 8.

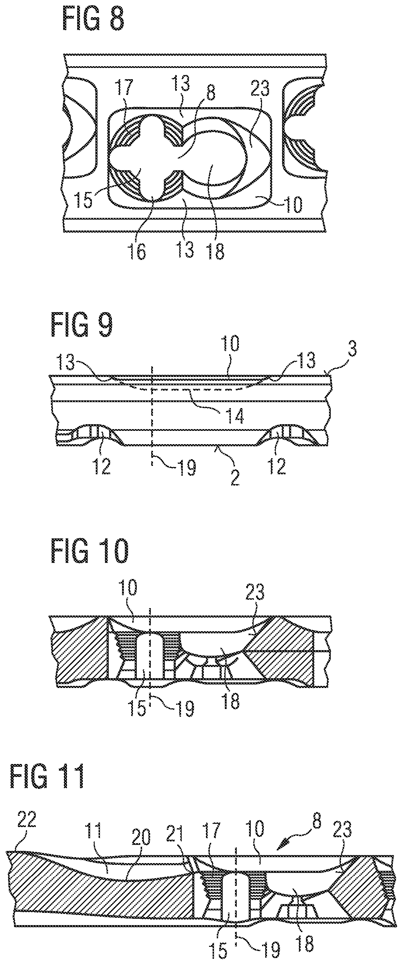

As represented in detail in Fig. 8, the through holes 8 in the shaft portion 4 are formed as combination holes. The through holes 8 have a variable-angle portion 15 as well as a compression hole 18. The variable-angle portion 15 possesses a rib- or thread-like structure 17 along an inner surface thereof, which tapers in a direction extending toward the bone-facing surface 2. The thread-like structure 17 is interrupted by recesses 16, which extend from the hole axis 19 radially outward. In the exemplary embodiment shown, there are formed four "columns" of the rib- or thread-like structure 17 which are separated from one another by the recesses 16. It will be understood by those of skill in the art, however, that the variable-angle portion 15 is not required to include four columns and may include, for example, 2 or more columns. As is known to the person skilled in the art, the variable-angle portion 15 is configured to receive a head locking screw which is introducible with its head thread into the variable-angle portion 15 of the through hole 8 at different angles, and lockable so that the screw is locked therewithin at a desired angle relative to the hole axis. The compression hole 18 has an unthreaded, i.e. smooth, inner surface 23 tapering in the direction of the bone-facing surface 2. The compression hole 18 is moreover elongated in the longitudinal direction of the shaft portion 4, so that the introduction of a compression screw with a smooth screw head therein causes a compression of the subjacent bone segments, as is known to the person skilled in the art.

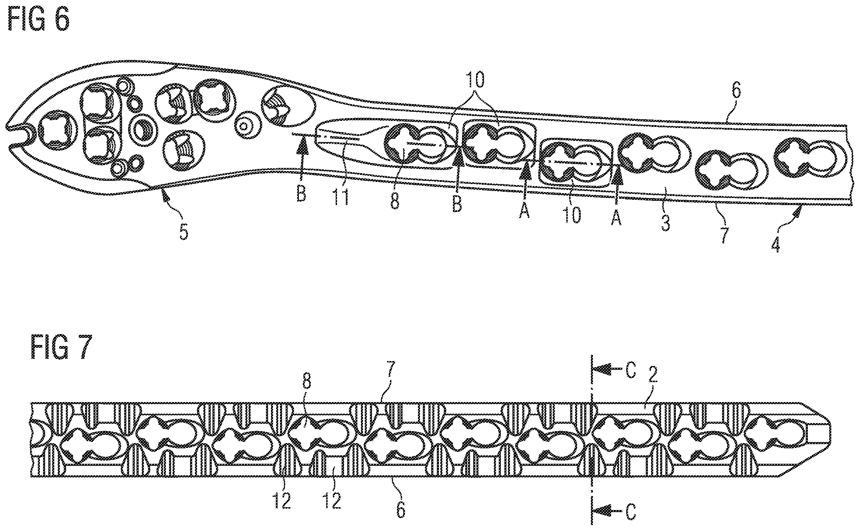

As is represented in Fig. 1, the first three through holes 8 along the shaft 4 that are closest in distance to the head portion 5, respectively overlap a depression 10 on the surface 3 of the plate's upper side. In other words, the depression 10 may extend through a portion of the bone plate 1 surrounding, or at least partially surrounding, the through hole 8. Although the exemplary embodiment specifically shows and describes three through holes 8 as including a depression 10 extending thereabout, it will be understood by those of skill in the art that any number of the through holes 8 may include a depression 10 extending thereabout. Furthermore, there is provided on the plate's upper side in the surface 3 a cavity 11 between the first through hole 8 - i.e., the through hole 8 closest in distance to the head portion 5 - and the head portion 5. On the second surface 2 there are arranged, as represented in Fig. 3, groups of recesses 12 along the length of the shaft portion 4. The depressions 10 and the cavity 11 are likewise represented in Figs. 4 and 6. The recesses 12 are seen in detail in Figs. 5 and 7. The depressions 10, the cavity 11 and the recesses 12 contribute both individually and in combination to reducing the risk of breakage of the bone plate 1, as described in detail hereinafter. Hence, it will be appreciated that the depressions 10, the cavity 11 and the recesses 12 can be provided in a bone plate separately or together.

Fig. 8 shows the depression 10 in plan view of the plate's first surface 3. The depression 10 may have a substantially rectangular shape and completely overlaps the through hole 8 formed as a combination hole. In particular in the region of the recesses 16 of the variable-angle portion 15 of the through hole 8, which point in the direction of the lateral side walls 6, 7 of the shaft portion 4 of the bone plate 1, the provision of the depression 10 can reduce stress peaks that occur when a bending load or torsional load is imposed on the bone plate 1. The mouth of the through hole 8 is brought closer to a center plane of the bone plate 1 by the depression 10. The depression 10 can be produced, for example, by milling the surface 3 of the bone plate 1. The depression 10 has edge regions 13 which, in the represented embodiment, overlap with the recesses 16 which point in the direction of the side walls 6, 7. In Fig. 9 the depression 10 is represented in a side view of the bone plate 1. A bottom portion 14 (e.g., a portion defining a depth of the depression 10) is indicated by dashed lines. The bottom portion 14 is substantially planar, whereas edge regions 13 are curved. The edge regions 13 pass into the bottom region 14 smoothly. Fig. 10 shows a cross-section of the through hole 8 and the depression 10 in a section along the line A-A in Fig. 6. Fig. 10 also shows the variable-angle portion 15 which overlaps with the compression hole 18.

In Fig. 11 shows a cross-section of the bone plate 1 along the line B-B in Fig. 6. The sectional representation shows the first through hole 8, closest in distance to the head portion 5, which overlaps with the depression 10. As also represented in Fig. 6, the cavity 11 borders with its distal end 21 on the depression 10 of the first through hole 8. The depression 10 and the cavity 11 are open to one another at the end 21 of the cavity 11. The depression 10 and the cavity 11 may have the same width, in particular, at the place where they border on each other. This enables the cavity 11 and the depression 10 to be produced, for example milled, in one step. The cavity 11 serves to increase the flexibility of the region between the first through hole 8 and the head portion 5 of the bone plate 1, so as to reduce the risk of breakage of the bone plate in the region of the first through hole 8. In particular, the bone fracture can lie in the region of the bone plate 1 that lies between the first through hole 8 and the head portion 5, so that an especially high load acts on the bone plate 1 here.

As represented in Fig. 11, the cavity 11 has a curved cross-sectional profile. The depth of the cavity 11 increases continuously from the shaft portion 4 in the direction of the head portion 5 from the distal end 21 of the cavity 11 to a deepest point 20. From the deepest point 20 to a proximal end 22 of the cavity 11 the depth of the cavity 11 decreases continuously, with the cavity 11 tapering off relatively flat, that is to say, enclosing an angle, which in one embodiment, is smaller than 10° relative to the first surface 3 of the bone plate 1. As seen in Figs. 4 and 6, the cavity 11 does not extend over the total width of the shaft portion 4 of the bone plate 1 (i.e., a distance between the lateral side walls 6, 7 of the bone plate), but is spaced from the side walls 6, 7. This retains a sufficient stability of the bone plate 1 in spite of the elevated flexibility caused by the cavity 11. The thicker edge regions absorb a greater part of a force flux than the bone plate 1 in the region of the cavity 11, so that the force flux is conducted around the first through hole 8 better than if the cavity 11 extended over the total width of the bone plate 1.

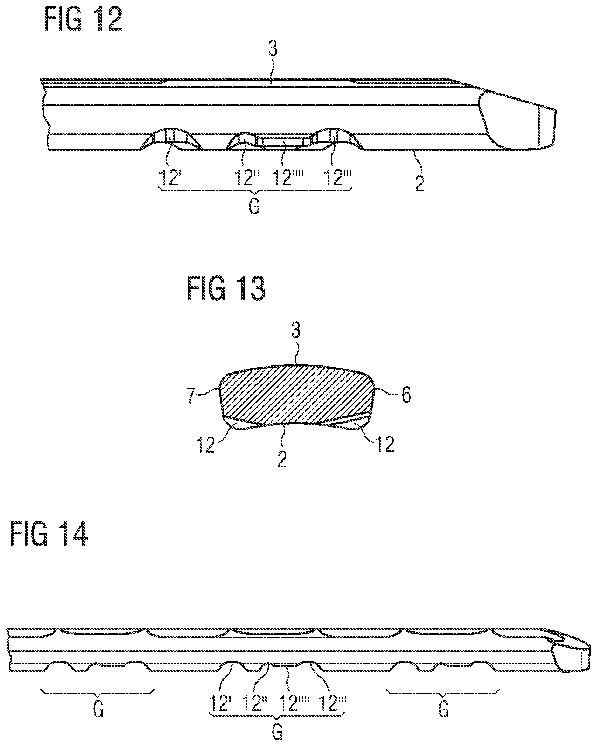

In Fig. 12 there is represented a side view of the bone plate 1, in particular of the shaft portion 4, in which a group G of recesses 12 is to be seen. For the sake of better representability, Fig. 14 shows the shaft portion 4 in a view rotated by 10° about the longitudinal axis of the bone plate 1. Fig. 13 shows a cross-sectional view of the shaft portion 4 of the bone plate 1 along the line C-C in Fig. 7. As seen in Figs. 7, 12 and 13, a group G of recesses 12 which is associated with one of the through holes 8 comprises four recesses 12', 12", 12"', 12"", whereby in this embodiment not only a plurality, but all groups G have the identical four recesses 12', 12", 12"', 12"" (except the first and the last group). In one exemplary embodiment, the first recess 12' and third recess 12"' are formed substantially identically, the second recess 12" being formed smaller. The fourth recess 12"" connects the second and the third recess 12", 12"', so that these can alternatively also be regarded as a contiguous recess. As seen in Fig. 14, the recesses 12 extend from one of the side walls 6, 7 obliquely toward the center of the bone plate 1, having the greatest depth on the side wall 6, 7 of the bone plate 1. As seen in particular in Fig. 7, the groups G of recesses 12 are arranged alternately offset from each other on the opposing side walls 6, 7 of the shaft portion 4 of the bone plate 1 and in the direction of longitudinal extension of the shaft portion 4, with the opposing groups G of recesses 12 partly overlapping in the direction of longitudinal extension of the shaft portion 4. The arrangement of the groups G of recesses 12 corresponds to the arrangement of the through holes 8 along the longitudinal extension of the shaft portion 4. As seen in Fig. 7, the through holes 8 are arranged offset from each other alternately with respect to a center axis extending in the direction of longitudinal extension of the shaft portion 4. Accordingly, the groups G of recesses 12 are also arranged offset from each other alternately with regard to the center axis. Preferably, the cross-sectional area can respectively be formed along the longitudinal extension of the shaft portion 4 such that the section modulus of the shaft portion 4 is substantially constant along its longitudinal extension, but at least uniform, i.e. not subject to any great fluctuations. This avoids the highest stress upon a bending of the bone plate 1 occurring at a through hole 8. As seen in Figs. 5 and 7, it will be appreciated that although the groups G of recesses 12 are preferably identical along the longitudinal extension of the shaft portion 4, the recesses 12 associated with the first through hole 8 (i.e., the through hole closest to the head portion 5) and the last through hole 8 (i.e., the through hole furthest from the head portion 5) are associated with a group G of recesses 12 which may not include all of the recesses 12', 12", 12"' and 12"" described above, because the respective end of the shaft portion 4 is reached.

Although the preferred embodiment was described with reference to a left or right proximal femur, it will be appreciated that the principle of the invention can also be applied to bone plates for other bones. For example, the invention can also be employed for the distal femur, the tibia or other load bearing long bones. It will be appreciated that the shape and dimensioning of the bone plate can be adapted in accordance with the case of application without impairing the principle of the invention.

It will be apparent to those skilled in the art that various modifications and variations may be made in the structure and the methodology of the present invention, without departing from the spirit or scope of the invention. Thus, it is intended that the present invention cover modifications and variations of the invention provided that they come within the scope of the appended claims and their equivalents.

## Claims

1. A bone plate (1) having a first, bone-facing surface (2) on a first side, and a second surface (3) on a second side opposing the first side, and extending along a longitudinal direction,
wherein the bone plate (1) has an elongate shaft portion (4) with two mutually opposing longitudinally running side edges (6, 7) which define a width of the shaft portion (4),
wherein a plurality of through holes (8) extend through the bone plate (1) from the first surface (2) to the second surface (3) and are configured to respectively receive a bone anchor,
wherein the first surface (2) has recesses (12) which extend from the opposing side edges (6, 7) over a part of the width of the shaft portion (4) transversely to the direction of longitudinal extension of the shaft portion (4), each of the plurality of through holes (8) having a plurality of the recesses (12) associated therewith, of which at least two recesses (12) differ from each other and which respectively form a group (G) of recesses (12), the groups (G) of recesses (12) being identical for each of the plurality of through holes (8),
**characterized in that** each group (G) of recesses (12) comprises a first, second and third recess (12', 12", 12"') which are arranged side by side in the longitudinal direction of the bone plate (1), the second recess (12") lying between the first and third recesses (12', 12"') and being formed smaller than the first and third recesses (12', 12"').

2. The bone plate according to claim 1, wherein each group (G) has a fourth recess (12"") which interconnects the second recess (12") with the first or third recess (12', 12"').

3. The bone plate according to claim 1 or 2, wherein the groups (G) of recesses (12) are arranged offset from each other on the opposing side edges (6, 7) of the bone plate (1) along the longitudinal direction of the bone plate (1).

4. The bone plate according to any of claims 1 to 3, wherein the groups (G) of recesses (12) extend alternately from the opposing side edges (6, 7).

5. The bone plate according to claim 4, wherein two groups (G) of recesses (12) following each other in the direction of longitudinal extension of the shaft portion (4) lie opposite each other on the opposing side edges (6, 7) such that their longitudinal extension intersects.

6. The bone plate according to any of claims 1 to 5, wherein the recesses (12) extend from the side edges (6, 7) at most up to a center axis of the bone plate (1) extending in the direction of longitudinal extension of the shaft portion (4).

7. The bone plate according to any of claims 1 to 6, wherein the bone plate (1) has a head portion (5) which is shorter and wider than the shaft portion (4), with the through holes (8) and the recesses (12) being arranged in the shaft portion (4).

8. The bone plate according to claim 7, wherein a total section modulus of the shaft portion (4) transversely to the direction of longitudinal extension of the shaft portion (4) is substantially constant along the direction of longitudinal extension of the shaft portion (4).

9. The bone plate according to claim 7 or 8, wherein the shaft portion (4) has along the direction of longitudinal extension of the shaft portion (4) a substantially constant section modulus in the direction of longitudinal extension.

10. The bone plate according to any of claims 1 to 9, wherein the recesses (12) possess a maximum depth on the side edges (6, 7).

11. The bone plate according to any of claims 1 to 10, wherein at least a respective one of the recesses (12) in the groups (G) of recesses (12) possesses a depth that decreases from the corresponding side edge (6, 7) in the direction of a center axis of the bone plate (1) extending in the direction of longitudinal extension of the shaft portion (4).

12. The bone plate according to any of claims 1 to 11, wherein the through holes (8) are formed as combination holes, having a variable-angle portion (15) which has an inner surface with a thread-like structure (17) which is interrupted by recesses (16), and a compression hole (18) overlapping with the variable-angle portion (15), which is formed elongately in the direction of longitudinal extension of the shaft portion (4) and has an unthreaded inner surface (23) tapering in the direction of the first, bone-facing surface (2).

13. The bone plate (1) according to claim 7 and any one of claims 1 to 6 and 8 to 12, wherein the second surface (3) has a cavity (11) which extends between that one of the through holes (8) situated closest to the head portion (5) and the head portion (5).

14. The bone plate according to claim 13, wherein the cavity (11) extends into the head portion (5).

15. The bone plate according to claim 13 or 14, wherein the second surface (3) has a depression (10) which overlaps the through hole (8) situated closest to the head portion (5), with the cavity (11) and the depression (10) bordering on each other and passing into each other.

## Patentansprüche

1. Knochenplatte (1) mit einer ersten, dem Knochen zugewandten Oberfläche (2) auf einer ersten Seite, und einer zweiten Oberfläche (3) auf einer zweiten Seite, die der ersten Seite gegenüberliegt, und sich entlang einer Längsrichtung erstreckt,
wobei die Knochenplatte (1) einen länglichen Schaftabschnitt (4) mit zwei einander gegenüberliegenden, in Längsrichtung verlaufenden Seitenrändern (6, 7) aufweist, die eine Breite des Schaftabschnitts (4) definieren,
wobei sich eine Vielzahl von Durchgangsöffnungen (8) durch die Knochenplatte (1) von der ersten Oberfläche (2) zur zweiten Oberfläche (3) erstreckt, die zum jeweiligen Aufnehmen eines Knochenankers konfiguriert sind,
wobei die erste Oberfläche (2) Ausnehmungen (12) aufweist, die sich von den gegenüberliegenden Seitenrändern (6, 7) über einen Teil der Breite des Schaftabschnitts (4) quer zur Längserstreckungsrichtung des Schaftabschnitts (4) erstrecken, wobei jede der Vielzahl von Durchgangsöffnungen (8) eine Vielzahl der dieser zugeordneten Ausnehmungen (12) aufweist, von denen sich zumindest zwei Ausnehmungen (12) voneinander unterscheiden und jeweils eine Gruppe (G) von Ausnehmungen (12) bilden, wobei die Gruppen (G) der Ausnehmungen (12) für jede der Vielzahl von Durchgangsöffnungen (8) identisch sind,
**dadurch gekennzeichnet, dass** jede Gruppe (G) von Ausnehmungen (12) eine erste, zweite und dritte Ausnehmung (12 ', 12 ", 12"') umfasst, die in Längsrichtung der Knochenplatte (1) nebeneinander angeordnet sind, wobei die zweite Ausnehmung (12") zwischen den ersten und dritten Ausnehmungen (12', 12"') liegt und kleiner als die ersten und dritten Ausnehmungen (12', 12"') ausgebildet ist.

2. Knochenplatte nach Anspruch 1, wobei jede Gruppe (G) eine vierte Ausnehmung (12"") aufweist, welche die zweite Ausnehmung (12") mit der ersten oder dritten Ausnehmung (12', 12"') verbindet.

3. Knochenplatte nach Anspruch 1 oder 2, wobei die Gruppen (G) von Ausnehmungen (12) an den gegenüberliegenden Seitenrändern (6, 7) der Knochenplatte (1) entlang der Längsrichtung versetzt zueinander angeordnet sind.

4. Knochenplatte nach einem der Ansprüche 1 bis 3, wobei sich die Gruppen (G) von Ausnehmungen (12) abwechselnd von den gegenüberliegenden Seitenrändern (6, 7) erstrecken.

5. Knochenplatte nach Anspruch 4, wobei sich zwei in der Längserstreckungsrichtung des Schaftabschnitts (4) aufeinanderfolgende Gruppen (G) von Ausnehmungen (12) an den gegenüberliegenden Seitenrändern (6, 7) einander so gegenüberliegen, dass sich deren Längserstreckung schneidet.

6. Knochenplatte nach einem der Ansprüche 1 bis 5, wobei sich die Ausnehmungen (12) von den Seitenrändern (6, 7) höchstens bis zu einer Mittelachse der Knochenplatte (1) erstrecken, die sich in Richtung der Längserstreckung des Schaftabschnitts (4) erstreckt.

7. Knochenplatte nach einem der Ansprüche 1 bis 6, wobei die Knochenplatte (1) einen Kopfabschnitt (5) aufweist, der kürzer und breiter als der Schaftabschnitt (4) ist, wobei die Durchgangsöffnungen (8) und die Ausnehmungen (12) im Schaftabschnitt (4) angeordnet sind.

8. Knochenplatte nach Anspruch 7, wobei ein Gesamt-Widerstandsmoment des Schaftabschnitts (4) quer zur Längserstreckungsrichtung des Schaftabschnitts (4) entlang der Längserstreckungsrichtung des Schaftabschnitts (4) im Wesentlichen konstant ist.

9. Knochenplatte nach Anspruch 7 oder 8, wobei der Schaftabschnitt (4) entlang der Längserstreckungsrichtung des Schaftabschnitts (4) ein im Wesentlichen konstantes Widerstandsmoment in der Längserstreckungsrichtung aufweist.

10. Knochenplatte nach einem der Ansprüche 1 bis 9, wobei die Ausnehmungen (12) an den Seitenrändern (6, 7) eine maximale Tiefe besitzen.

11. Knochenplatte nach einem der Ansprüche 1 bis 10, wobei zumindest eine jeweilige der Ausnehmungen (12) in den Gruppen (G) von Ausnehmungen (12) eine Tiefe aufweist, die vom entsprechenden Seitenrand (6, 7) in Richtung einer Mittelachse der Knochenplatte (1) abnimmt, die sich in der Längserstreckungsrichtung des Schaftabschnitts (4) erstreckt.

12. Knochenplatte nach einem der Ansprüche 1 bis 11, wobei die Durchgangsöffnungen (8) als Kombinationsöffnungen ausgebildet sind, die einen Abschnitt (15) mit variablem Winkel aufweisen, der eine Innenfläche mit einer gewindeartigen Struktur (17), die durch Ausnehmungen (16) unterbrochen ist, und eine Kompressionsöffnung (18) aufweist, die den Abschnitt (15) mit variablem Winkel überlappt, der in der Längserstreckungsrichtung des Schaftabschnitts (4) länglich ausgebildet ist und eine Innenfläche (23) ohne Gewinde aufweist, die sich in der Richtung der ersten, dem Knochen zugewandten Oberfläche (2) verjüngt.

13. Knochenplatte (1) nach Anspruch 7 und einem der Ansprüche 1 bis 6 und 8 bis 12, wobei die zweite Oberfläche (3) einen Hohlraum (11) aufweist, der sich zwischen der einen der Durchgangsöffnungen (8) erstreckt, die dem Kopfabschnitt (5) und dem Kopfabschnitt (5) am nächsten liegt.

14. Knochenplatte nach Anspruch 13, wobei sich der Hohlraum (11) in den Kopfabschnitt (5) erstreckt.

15. Knochenplatte nach Anspruch 13 oder 14, wobei die zweite Oberfläche (3) eine Vertiefung (10) aufweist, welche die Durchgangsöffnung (8), die dem Kopfabschnitt (5) am nächsten liegt, mit dem Hohlraum (11) und der Vertiefung (10) überlappt, die aneinander grenzen und ineinander übergehen.

## Revendications

1. Lame osseuse (1), présentant une première surface faisant face à l'os (2) sur un premier côté, et une seconde surface (3) sur un second côté opposé au premier côté, et s'étendant le long d'une direction longitudinale,
dans laquelle la lame osseuse (1) présente une portion de tige allongée (4) avec deux bords latéraux orientés longitudinalement et opposés l'un à l'autre (6,7), lesquels définissent une largeur de la partie de tige (4) ;
dans laquelle une pluralité de trous débouchants (8) s'étendent à travers la lame osseuse (1) de la première surface (2) à la seconde surface (3) et sont configurés pour recevoir respectivement un ancrage osseux ;
dans laquelle la première surface (2) présente des retraits (12), lesquels s'étendent des bords latéraux opposés (6,7) par-dessus une partie de la largeur de la portion de tige (4) transversalement à la direction d'extension longitudinale de la portion de tige (4), chacun de la pluralité de trous débouchants (8) présentant une pluralité des retraits (12) associés à ceux-ci, parmi lesquels au moins deux retraits (12) diffèrent les uns des autres, et lesquels forment respectivement un groupe (G) de retraits (12), les groupes (G) de retraits (12) étant identiques pour chacun de la pluralité de trous débouchants (8),
**caractérisée en ce que** chaque groupe (G) de retraits (12) comprend un premier, un deuxième et un troisième retraits (12', 12", 12"'), lesquels sont agencés côte à côte dans la direction longitudinale de la lame osseuse (1), le deuxième retrait (12") se trouvant entre les premier et troisième retraits (12', 12"') et étant formé de manière à être plus petit que les premier et troisième retraits (12', 12"').

2. Lame osseuse selon la revendication 1, dans laquelle chaque groupe (G) présente un quatrième retrait (12""), lequel relie le deuxième retrait (12") avec le premier ou le troisième retraits (12', 12"').

3. Lame osseuse selon la revendication 1 ou 2, dans laquelle les groupes (G) de retraits (12) sont agencés de manière à être décalés les uns par rapport aux autres sur les bords latéraux opposés (6, 7) de la lame osseuse (1) le long de la direction longitudinale de la lame osseuse (1).

4. Lame osseuse selon l'une quelconque des revendications 1 à 3, dans laquelle les groupes (G) de retraits (12) s'étendent de manière alternée à partir des bords latéraux opposés (6, 7).

5. Lame osseuse selon la revendication 4, dans laquelle deux groupes (G) de retraits (12) se suivant entre eux dans la direction d'extension longitudinale de la portion de tige (4) se trouvent en opposition les uns aux autres sur les bords latéraux opposés (6, 7) de telle sorte que leur extension longitudinale forme une intersection.

6. Lame osseuse selon l'une quelconque des revendications 1 à 5, dans laquelle les retraits (12) s'étendent à partir des bords latéraux opposés (6, 7) au maximum jusqu'à un axe central de la lame osseuse (1) s'étendant dans la direction d'extension longitudinale de la portion de tige (4).

7. Lame osseuse selon l'une quelconque des revendications 1 à 6, dans laquelle la lame osseuse (1) présente une portion de tête (5), laquelle est plus courte et plus large que la portion de tige (4), les trous débouchants (8) et les retraits (12) étant agencés dans la portion de tige (4).

8. Lame osseuse selon la revendication 7, dans laquelle un module de section total de la portion de tige (4) transversalement à la direction d'extension longitudinale de la portion de tige (4) est en grande partie constant le long de la direction d'extension longitudinale de la portion de tige (4).

9. Lame osseuse selon la revendication 7 ou 8, dans laquelle la portion de tige (4) présente, le long de la direction d'extension longitudinale de la portion de tige (4), un module de section en grande partie constant dans la direction d'extension longitudinale.

10. Lame osseuse selon l'une quelconque des revendications 1 à 9, dans laquelle les retraits (12) présentent une profondeur maximale sur les bords latéraux (6,7).

11. Lame osseuse selon l'une quelconque des revendications 1 à 10, dans laquelle au moins un retrait respectif parmi les retraits (12) dans les groupes (G) de retraits (12) présente une profondeur qui diminue à partir du bord latéral correspondant (6, 7) dans la direction d'un axe central de la lame osseuse (1) s'étendant dans la direction d'extension longitudinale de la portion de tige (4).

12. Lame osseuse selon l'une quelconque des revendications 1 à 11, dans laquelle les trous débouchants (8) sont formés sous la forme de trous de combinaison, présentant une portion à angle variable (15), laquelle présente une surface intérieure avec une structure de type filetée (17) interrompue par des retraits (16), et un trou de compression (18) présentant un chevauchement avec la portion à angle variable (15), lequel est formé de manière allongée dans la direction d'extension longitudinale de la portion de tige (4) et présente une surface intérieure non filetée (23) à amincissement progressif dans la direction de la première surface faisant face à l'os (2).

13. Lame osseuse selon la revendication 7 et selon l'une quelconque des revendications 1 à 6 et 8 à 12, dans laquelle la seconde surface (3) présente une cavité (11), laquelle s'étend entre le trou parmi les trous débouchants (8) situé le plus près de la portion de tête (5) et la portion de tête (5).

14. Lame osseuse selon la revendication 13, dans laquelle la cavité (11) s'étend jusque dans la portion de tête (5).

15. Lame osseuse selon la revendication 13 ou 14, dans laquelle la seconde surface (3) présente un creux (10), lequel chevauche le trou débouchant (8) situé le plus près de la portion de tête (5), la cavité (11) et le creux (10) étant en bordure l'un de l'autre et passant dans l'un et l'autre.
